# FASCICULE DE BREVET EUROPEEN

(11) **EP 2 010 012 B1**
(45) Date de publication et mention de la délivrance du brevet: **30.11.2016**
(21) Numéro de dépôt: 07731932.5
(22) Date de dépôt: 23.04.2007
(51) Int. Cl.: A23L 27/10, A23L 33/105, A61K 31/11, A61K 31/351, A61K 31/7028, A61K 36/88, A61P 3/04

(54) **UTILISATION D'UN EXTRAIT DE SAFRAN EN TANT QU'AGENT DE SATIÉTÉ POUR LE TRAITEMENT DE LA SURCHARGE PONDÉRALE**
VERWENDUNG EINES SAFRANEXTRAKTS ALS SÄTTIGUNGSMITTEL FÜR DIE BEHANDLUNG VON ÜBERGEWICHT
USE OF AN EXTRACT OF SAFFRON AS SATIETY AGENT FOR THE TREATMENT OF OVERWEIGHT

(30) Priorité: 25.04.2006 FR 0651443
(43) Date de publication de la demande: 07.01.2009
(73) Titulaire: Bourges, Cédric, 22190 Plerin (FR)
(72) Inventeur: Bourges, Cédric, 22190 Plerin (FR)
(74) Mandataire: Laget, Jean-Loup
(86) Numéro de dépôt international: PCT/FR2007/051158
(87) Numéro de publication internationale: WO 2007/125243

(56) Documents cités:
- EP-A1- 1 481 675
- JP-A- 2003 206 225
- DATABASE WPI Week 200374 Derwent Publications Ltd., London, GB; AN 2003-783251 XP002414278 & JP 2003 206225 A (ICHIMARU PHARCOS INC) 22 juillet 2003 (2003-07-22)

## Description

La présente invention concerne l'utilisation des principes actifs du safran, en particulier le safranal, la crocine, la picrocrocine pour une application en tant qu'agent de satiété.

L'utilisation d'un agent de satiété est utile notamment pour le traitement de l'obésité, pour réduire ou au moins contrôler l'absorption de calories consommées au quotidien afin de réguler le poids corporel. Ainsi, l'utilisation d'un agent de satiété va permettre au consommateur de par l'altération du sens de la satiété, de contrôler son apparence physique et/ou de traiter ses problèmes de santé liés à surcharge pondérale.

Une augmentation pondérale entraîne en effet, en plus des désagréments moraux (problèmes psychologiques) liés aux dictats de la minceur et de la beauté des sociétés modernes, des problèmes de santé. Les conséquences des problèmes de poids et de l'obésité peuvent aller d'une augmentation de la mortalité précoce à des troubles non fatals mais débilitants, ayant des effets pervers sur la qualité de vie. Les principaux problèmes de santé associés à l'obésité et la surcharge pondérale sont : le diabète de type 2, les maladies cardiovasculaires et l'hypertension, les maladies respiratoires (le syndrome d'apnée du sommeil), certains cancers et l'ostéo-arthrite. A ceci s'ajoute une diminution dans la perception de la qualité de vie.

Divers facteurs favorisent l'augmentation de la surcharge pondérale.

Le mode d'alimentation actuel est appauvri en légumes et en fruits, mais par contre très riche en graisse, excessif en sucre, en viande et en alcool. De plus, la consommation d'eau est progressivement remplacée par la consommation de boissons gazeuses sucrées. Cette malnutrition alimentaire entraîne une augmentation du nombre de calories consommées quotidiennement et ainsi une éventuelle prise de poids.

De plus, certains comportements dans le mode de vie actuel, tels que l'insuffisance d'activité physique, les changements d'habitudes fréquents des individus contribuent davantage au risque de surcharge pondérale.

En outre, la prise de certains médicaments usuellement utilisés pour traiter des affections courantes, tels que la cortisone en tant qu'anti-inflammatoire ou le citalopram, l'imipramine, la paroxétine, la cyamémazine et le bromazépam en tant qu'anti-dépresseurs provoque une prise de poids importante.

Or, il n'est toujours pas évident pour une personne présentant une surcharge pondérale de suivre un régime particulier, du fait notamment des contraintes que cela exige et de la difficulté de l'adapter au mode de vie actuel.

De nombreuses solutions ont ainsi été proposées afin d'aider les individus à contrôler leur poids.

Par exemple, l'utilisation de médicaments supprimant l'activité des enzymes dans le système digestif. Toutefois, l'utilisation de tels médicaments n'est requise que dans un but médical.

Une solution invasive consiste à la mise en place d'un anneau gastrique afin de séparer l'estomac en deux parties. Si cette solution entraîne la disparition de la faim dès l'ingestion des premières bouchées d'un repas, il ne reste pas moins que cette solution est utilisée dans des cas de surcharge pondérale extrême. En outre, le risque de cette opération chirurgicale n'est pas négligeable.

D'autres produits ont été développés pour induire la perte de poids, comme les barres ou boissons de substitution alimentaire, le plus souvent à base de fibres alimentaires, de protéines, de carboxyméthyl-cellulose et de gommes. Toutefois, les produits de substitution ne permettent pas de combler la sensation de faim jusqu'au prochain repas, entraînant dès lors un risque de grignotage pour le consommateur afin de combler cette faim.

Une autre approche dans le traitement de la surcharge pondérale est l'utilisation d'agents de satiété afin de réduire l'apport énergétique, et donc le poids.

Ces derniers sont le plus souvent contenus dans le produit alimentaire à consommer.

Certains de ces agents de satiété sont relâchés directement dans l'estomac (WO 02/00042), d'autres agissent au niveau des différentes parties de l'intestin afin de modifier la réponse du frein iléal (US 6,267,988, US 5,753,253, DE 2701361) ou encore certains augmentent la sécrétion de l'a-MSH par inhibition de la recapture de la dopamine ou de la sérotonine.

Le document WO 01/17541 divulgue une composition comprenant des protéines, un niveau élevé de calcium, des acides gras à longue chaîne, une source d'inhibiteur de la protéase extraite de la pomme de terre afin d'induire la satiété.

Le document WO 01/17377 expose des polysaccharides contenant de l'acide uronique réticulés les uns aux autres afin de former une structure de type éponge qui se dissout mal dans l'eau, dans les fluides de l'estomac et de l'intestin ou peut être mal résorbée. Cette composition a pour effet de fournir un effet de satiété.

US 2006/0040003 décrit une composition pour améliorer la satiété avant et après le repas en élevant le taux de sérotonine. Cette composition comprend des substances naturelles comme, entre autres, de la niacine, de la vitamine B6, du calcium, du phosphore, du magnésium, du chitosan, du ginseng.

EP 1481675 décrit un agent pour élever la température du corps et obtenir des effets anti-obésité. Cet agent comprend un acide gras en C₆-C₁₂ et/ou un ester d'acide gras de glycérine incluant un tel acide gras.

Toutefois, les compositions telles que décrites dans l'état de la technique présentent divers inconvénients : la sensation de satiété n'est pas toujours de longue durée. Les préparations alimentaires (soupes, barres) contenant les produits sont généralement peu appétissantes pour le consommateur. En outre, ces compositions ne sont pas toujours aisées à fabriquer, certaines ne sont en effet pas stables pendant le procédé de fabrication et/ou le stockage, altérant le goût, l'odeur et la texture du produit final. De plus, elles peuvent selon les cas avoir éventuellement des interactions dommageables avec d'autres substances actives telles que les vitamines, ou les minéraux. Par ailleurs, les produits contenant des composés chimiques de synthèse, tels que des polymères, rebutent le consommateur à l'achat. C'est pourquoi les composés naturels sont préférés.

On connaît de JP 2002-0004234 un activateur de l'acide glutamique décarboxylase comprenant des agents actifs issus des plantes suivantes : *Aloe arborescens, Aloe vera, Cassia tora, Cassia obtusifolia,* amande, laurier, anis, safran, gingembre, *Cuminum cyminum et* poivre de Cayenne.

Le safran est connu dans l'état de la technique uniquement en tant que stimulateur de la fonction gastrique, régulateur d'âcreté, notamment après l'absorption de poivrons (JP 2005 143308) ou encore il est utilisé afin d'améliorer l'arôme dans les compositions de parfum (EP 0162465).

L'invention a pour but de proposer une nouvelle utilisation du safran et de ses principes actifs qui présentent de nombreuses qualités et qui permettent d'éviter tout ou partie des inconvénients précités. En particulier, l'invention a pour but de provoquer une sensation de satiété chez la personne ayant une surcharge pondérale, voire de traiter efficacement les problèmes de poids sans effets secondaires néfastes sur l'organisme, et sans que le consommateur ait la nécessité de modifier son mode de vie ou son régime alimentaire.

A cet effet, l'invention concerne l'utilisation d'un extrait de safran contenant du safranal, de la picrocrocine et de la crocine dans la production d'un agent de satiété actif pour le traitement des problèmes de santé liés à la surcharge pondérale.

L'invention concerne également l'utilisation d'un extrait de safran contenant du safranal, de la picrocrocine et de la crocine dans la production d'un agent de satiété actif pour le traitement des troubles alimentaires, des envies compulsives de grignotage liées à la dépression.

L'invention a encore pour objet l'utilisation d'un extrait de safran contenant du safranal, de la picrocrocine et de la crocine pour la fabrication d'un agent de satiété actif pour le traitement de l'obésité.

Un autre but de l'invention concerne l'utilisation non thérapeutique d'un extrait de safran contenant du safranal, de la picrocrocine et de la crocine en tant qu'agent de satiété actif pour aider l'individu présentant une surcharge pondérale à réduire ou contrôler son absorption de calories au quotidien et/ou contrôler son poids corporel et/ou son apparence physique.

L'invention a encore pour objet l'utilisation d'un extrait de safran contenant du safranal, de la picrocrocine et de la crocine, dans laquelle ce ou ces principes actifs sont associés à au moins un excipient ou véhicule inerte, non toxique, pharmaceutiquement et/ou alimentairement acceptable permettant son administration par voie orale.

Un autre but de l'invention est l'utilisation d'un extrait de safran contenant du safranal, de la picrocrocine et de la crocine, dans laquelle ce ou ces principes actifs se présentent sous forme d'une solution ou d'une suspension aqueuse dans une ampoule en verre, en boisson, en flacon compte-gouttes, en spray, ou à l'état sec de comprimés nus ou enrobés, de gélules, de capsules, de poudres, de comprimés effervescents, de granules, de strips, de pastilles.

Généralement, la teneur en safranal, picrocrocine, crocine est choisie pour une administration s'échelonnant entre 0,05 mg/jour à 100 mg/jour, de préférence entre 0,2 mg/jour et 10 mg/jour.

De préférence, la teneur du safranal, de la picrocrocine et de la crocine au sein de l'agent de satiété est comprise entre 0,01 et 50 %, de préférence entre 0,05 et 20 %, et plus particulièrement entre 0,1 et 10 %, en poids, par rapport au poids total de l'agent de satiété lorsque celui-ci se trouve sous forme solide ou par rapport au volume de l'agent de satiété lorsque celui-ci se trouve sous forme liquide.

Avantageusement, les principes actifs du safran : safranal, picrocrocine et crocine, sont extraits par hydrodistillation suivie d'extraction liquide/liquide par des solvants organiques polaires et apolaires ou par une hydrodistillation sous vide par micro-ondes (VMHD) ou encore par une extraction au CO₂ supercritique, ou aux ultrasons, ou aux résines échangeuses d'ions avec des co-solvants et solvants d'élution polaires et/ou apolaires, ou encore par le jeu d'extraction solides/liquides suivies d'extractions liquides/liquides.

L'invention sera mieux comprise, et d'autres buts, détails, caractéristiques et avantages de celle-ci apparaitront plus clairement au cours de la description explicative détaillée qui va suivre, de modes de réalisation de l'invention donnés à titre d'exemples purement illustratifs et non limitatifs, en référence aux figures annexées dans lesquelles :
- la figure 1 représente l'évolution moyenne du sentiment de faim avant le repas de 16 sujets ayant soit reçu un traitement placebo pendant 28 jours (groupe 1), soit une composition comprenant un extrait de stigmate de safran selon la présente invention (groupe 2) entre la fin (J28) et le début (J0) de l'expérimentation ;
- la figure 2 représente l'évolution moyenne du sentiment de faim du groupe 1 et du groupe 2 en fin de repas au cours de l'expérimentation de J0 à J28 ;
- la figure 3 montre la diminution de la masse corporelle en poids, en masse grasse et en masse maigre du groupe 1 et du groupe 2 de J0 à J28;

Tel qu'indiqué précédemment, il a été trouvé de manière surprenante que les principes actifs de safran tel que le safranal (2,6,6-triméthyl-1,3-cyclohexadiène-1-carboxaldéhyde) de formule suivante : 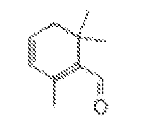 la picrocrocine (4-(béta-D-glucopyranosyloxy)-2,6,6-triméthyl-1 cyclohexane-1-carboxaldéhyde) et la crocine (Bis (6-0-béta-D-glucopyranosyl-béta-D-glycopyranosyl)8,8'diapa.psi.,psi.carotènedioate) de formule suivante : 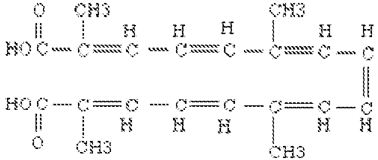 se sont avérés efficaces dans le traitement des problèmes de poids des individus présentant une surcharge pondérale.

D'après l'Organisation Mondiale de la Santé, une personne présente une surcharge pondérale lorsque son Indice de Masse Corporelle (IMC), qui est égal au poids en kg/(taille en mètre)² se situe entre 25,0 et 29,9. Si cet indice se situe au dessus de 30,0, la personne est considérée comme obèse. Cet indice permet ainsi d'estimer l'état nutritionnel d'une personne et de connaître si cette personne ne présente pas un excès de graisse dans le corps.

Les principes actifs du safran sont extraits d'une plante, le *Safran Crocus sativus L*., et plus particulièrement des stigmates de cette plante. Il est également possible d'extraire ces principes actifs d'autres plantes, telles que *Yucca periculosa*, *Ditaxis heterantha, Cuminum cyminum, Gardenia jasminoides* ou *Camelia Sinensis.*

Le procédé utilisé est connu de l'homme du métier. Il s'agit d'une première étape d'hydrodistillation, suivie ensuite d'une seconde étape d'extraction liquide/liquide.

L'hydrodistillation sert à extraire l'huile essentielle de la plante non miscible à l'eau. L'essence est entraînée par la vapeur sous la forme d'un hétéroazéotrope. La température d'ébullition du mélange est inférieure à 100°. Un mélange de substances organiques et d'eau est ainsi récupéré. Il est également possible d'utiliser l'hydrodistillation par micro-ondes sous vide pulsé (VHMD).

L'extraction liquide/liquide est réalisée par le contact intime du solvant avec la solution dans des appareils destinés à mélanger les deux phases (ampoules, colonnes, mélangeurs). La séparation des phases s'obtient par décantation gravimétrique ou centrifuge. Les solvants utilisés pour l'extraction sont l'eau et l'éthanol ou encore l'acétate d'éthyle, l'hexane, l'éther de pétrole, l'acétone ou le méthanol.

L'extraction du safranal, ainsi que des autres principes actifs naturels du safran peut également se faire à l'aide du CO₂ supercritique, idéalement à 100°C et 20 MPa.

Un produit liquide sirupeux, de couleur rouge grenat profond avec des reflets irisés, est obtenu à température ambiante. Il peut également se présenter sous forme de poudre après évaporation de l'eau, par exemple après étuvage ou atomisation.

Les principes actifs ainsi extraits : la picrocrocine, la safranal et la crocine répondent en outre aux normes ISO 3632.

L'agent de satiété comprend une teneur en safranal et/ou de la picrocrocine et/ou de la crocine et/ou de leurs dérivés entre 0,01 et 50 %, mais de préférence une teneur entre 0,1 et 10 % et plus particulièrement de 1 à 5%, en poids, par rapport au poids total de l'agent de satiété lorsque celui-ci se trouve sous forme solide ou liquide..

D'autres agents pharmaceutiquement et/ou alimentairement acceptables peuvent être rajoutés à l'agent de satiété, tels que des agents de charge, des fluidisants, des extraits naturels, des vitamines, des minéraux, des oligoéléments, des acides aminés, des acides gras, des anti-agglomérants, des huiles naturelles, des arômes, des colorants, des acidifiants, des épaississants, des conservateurs et des édulcorants.

Les charges sont avantageusement de la cellulose microcristalline, de la maltodextrine de pomme de terre et le lactate de magésium.

L'épaississant utilisé de préférence est l'amidon de pomme de terre, de l'hydroxypropylméthylcellulose, la pectine de citrus, la gomme de guar, de caroube, agar-agar, konjac, les huiles hydrogénées, la cire d'abeille.

Les fluidisants peuvent être du silicate de magnésium, du stéarate de magnésium, de la silice colloïdale.

Les anti-agglomérants sont ceux utilisés usuellement dans l'industrie alimentaire, tels que le stéarate de magnésium, la silice colloïdale.

Les vitamines sont choisies parmi, entre autres, les vitamines C, E, B₆, B₁, B₂, B₃.

Les extraits naturels en plus de l'extrait de stigmates de safran peuvent être des extraits de thé vert, de cannelle, de guarana, de maté, de fenouil, de reine des prés, de maïs, de sauge, de mélisse et de la caféine.

En tant qu'acidifiant, de l'acide citrique peut être utilisé dans la composition de l'agent de satiété.

Les stabilisants employés dans la fabrication de l'agent de satiété sont ceux usuellement utilisés dans l'industrie agro-alimentaire, tels que le sorbitol.

Les arômes qui peuvent être utilisés sont variés, tels que l'arôme de café, de citron, de pomme, de chocolat, de vanille, de fraise.

Les édulcorants utilisés sont, entre autres, le xylitol, l'aspartame, le sirop de glucose, le sirop de fructo-oligosaccharide, le maltitol en poudre ou en sirop, l'acésulfame de potassium, le fructooligosaccharide et le cyclamate de sodium.

Des acides gras peuvent également être ajoutés à l'agent de satiété, tels que oméga 3, oméga 6, Galacto, lipides, mais aussi des minéraux : chrome, bore, magnésium, calcium, fer, molybdène et des acides aminés, tels que tryptophane, leucine, arginine, et glycine.

Les conservateurs sont utiles afin que l'agent de satiété se conserve dans le temps. Les conservateurs utilisés peuvent être par exemple le sorbate de potassium, les parabènes, le benzoate de sodium ou le palmitate d'ascorbyle (anti-oxydant).

Tous ces composés ne sont nullement limitatifs des agents pharmaceutiquement et alimentairement acceptables pouvant être ajoutés à la composition de l'agent de satiété pour le traitement de la surcharge pondérale.

L'agent de satiété comprenant entre autres les principes actifs du safran présente l'avantage d'être facilement utilisable et non contraignant. En outre, comme les résultats ci-dessous l'indiquent, la teneur en principes actifs du safran et plus particulièrement en safranal n'a pas besoin d'être très élevée afin d'obtenir l'effet recherché, c'est-à-dire un effet de satiété et donc une perte de poids de masse grasse, de tour de taille et de hanches assez rapide. Contrairement, aux agents de satiété de l'état de l'art, l'extrait de stigmates de safran pris en ampoule ou en cachet ne présente pas d'odeur, de texture ou de goût désagréables pour l'utilisateur. En outre, il suffit de prendre une ou deux doses journalières, le plus souvent au petit déjeuner et/ou déjeuner afin de contrôler ou réduire l'absorption de calories au cours de la journée. Par ailleurs, comme indiqué plus haut, l'extrait de stigmates de safran peut être associé à d'autres principes actifs, tels que des vitamines, sans que cela n'interagisse sur l'efficacité du principe actif. Enfin, l'agent de satiété ainsi obtenu est stable lors de sa fabrication et de son stockage.

L'utilisation de l'extrait du safran pour aider l'individu présentant une surcharge pondérale à réduire ou contrôler son absorption de calories au quotidien et/ou contrôler son poids corporel et/ou son apparence physique, peut se faire de différentes manières. En effet, ce principe actif peut être tout simplement ajouté à tout aliment de consommation courante (boissons, repas préparés...), ou bien être sous la forme de complément alimentaire, de produits diététiques, de dispositif médical, de médicaments. Dans ce dernier cas, la concentration en extrait de stigmates de safran au sein du médicament sera augmentée afin que l'effet de satiété se fasse davantage ressentir. Par exemple, une dose en extrait de stigmates de safran allant de 60 à 150 mg par médicament conviendra particulièrement aux personnes atteintes de la maladie de l'obésité.

Les formulations suivantes (tableau 1 à 8) sont données à titre d'exemples purement illustratifs.
1) Comprimé ovale pelliculé taille 12

**Tableau 1**

| **Matières** | **Dose en mg** |
|---|---|
| **COMPRIMÉ NU :** | |
| Agent de charge : cellulose microcristalline | 150,00 |
| Lactate de magnésium | 254,00 |
| Extrait de stigmates de safran > 2% safranal | 30,00 |
| Vitamine B6 | 2,00 |
| Extrait de mélisse | 10,00 |
| Fluidisant : silicate de magnésium | 6,00 |
| Amidon de pomme de terre | 6,00 |
| Stéarate de magnésium | 5,00 |
| Silice colloïde | 2,00 |
| **TOTAL NU** | **465,00** |
| **PELLICULAGE** | **18,60** |
| Sépifilm LP014 | 11 ,16 |
| Sepisperse dry 5221 | 7,44 |
| **TOTAL PÉLLICULE** | **502,20** |

2) Gélule taille 1, tunique d'origine végétale

**Tableau 2**

| **Ingrédients** | **Dose (mg)** |
|---|---|
| Agent de charge : maltodextrine de pomme de terre | 80,00 |
| Gélule HPMC | 80,00 |
| Extrait de thé vert riche en EGCG | 60,00 |
| Extrait de cannelle écorce | 50,00 |
| Extrait de stigmates de safran > 2 % safranal | 20,00 |
| Poudre de stigmate de safran | 20,00 |
| Vitamine B3 | 18,00 |
| Chlorure de chrome | 0,08 |
| Vitamine E sous forme poudre à 50 % (calcul suivant forme DL alpha tocophérol à 50 %) | 20,00 |
| Agent de charge : cellulose MCC | 10,00 |
| Anti-aglomérant : stéarate de magnésium | 10,00 |
| Fluidifiant : silice colloidale | 5,00 |
| **TOTAL** | **373,08** |

3) Capsule molle

**Tableau 3**

| **Ingrédients** | **mg/capsule** |
|---|---|
| Huile de poissons sauvages à 30 % d'acides gras polyinsaturés oméga 3 18 % EPA 12 % DHA | 577,00 |
| Extrait de safran > 2 % safranal | 40,00 |
| Gélatine de poisson | 130,07 |
| Glycérol | 61,00 |
| Arôme citron | 1,93 |
| **TOTAL** | **810,00** |

4) Comprimé effervescent

**Tableau 4**

| **Ingrédients** | **mg ingrédient/comprimé** |
|---|---|
| Acidifiant : acide citrique (E330) | 1070,000 |
| Bicarbonate de sodium | 727,000 |
| Extrait de stigmates de safran > 2 % safranal | 60,000 |
| Extrait de thé vert feuille 20-30 % de catéchines | 220,000 |
| Stabilisant : sorbitol | 146,000 |
| Vitamine C | 72,000 |
| Extrait de guarana 12 % caféine | 70,000 |
| Arôme poudre citron vert | 70,000 |
| Extrait de maté 8 % de caféine | 42,500 |
| Edulcorant : aspartame | 30,000 |
| Colorant jaune : tartrazine (E102) | 0,210 |
| Chlorure de chrome (19 % de chrome) | 0,025 |
| **TOTAL** | **2507,735** |

5) Ampoule verre de 10 Ml

**Tableau 5**

| **Ingrédients** | **g/ampoule** |
|---|---|
| Extrait aqueux concentré par macération à chaud d'un mélange de plantes : | 9,97 |
| - Fenouil - bulbe - | 20% |
| - Reine des prés - sommité fleurie- | 20% |
| - Maïs - stigmates - | 20% |
| - Thé vert - feuille - | 20% |
| - Guarana - noix- | 20% |
| Extrait de stigmates de safran > 2 % safranal | 0,03 |
| **TOTAL** | **10,00** |

6) Boisson

**Tableau 6**

| **Ingrédients** | **mg/50 mL** |
|---|---|
| Eau | qsp 50 mL |
| Extrait liquide de stigmate de safran > 2 % safranal | 50,00 |
| arôme café | 146,40 |
| Acide citrique | 111,44 |
| Caféine naturelle de café | 96,00 |
| Extrait de feuille de thé vert | 51,00 |
| Sorbate de potassium | 50,00 |
| Benzoate de sodium | 50,00 |
| Acésulfame de postassium | 3,76 |
| Cyclamate de sodium | 3,76 |
| **TOTAL pour 50 mL** | **50 000,00** |

7) Carré

**Tableau 7**

| **Ingrédients** | **g/carré** |
|---|---|
| Pectine de citrus | 3,600 |
| Gomme guar | 2,258 |
| Sirop de glucose | 1,660 |
| Sirop de fructo-oligossacharide | 0,640 |
| Sirop de maltitol | 0,670 |
| Maltitol poudre | 0,500 |
| Arôme pomme | 0,200 |
| Glycérol poudre | 0,190 |
| Huile TCM | 0,136 |
| Eau | 0,100 |
| Extrait liquide de stigmates de safran > 2 % safranal | 0,02 |
| Acide citrique | 0,011 |
| Tocophérol de soja (67-75%) | 0,003 |
| Palmitate d'ascorbyle | 0,001 |
| **TOTAL/CARRE** | **10,000** |

8) Granule

**Tableau 8**

| **Ingrédients** | **g/pour 100 g de granulés** |
|---|---|
| Pectine de citrus | 75,00 |
| Cellulose micro cristalline | 20,00 |
| Sorbitol | 4,97 |
| Extrait de stigmates de safran > 2% safranal | 0,03 |
| **TOTAL** | **100,00** |

Des tests expérimentaux ont été réalisés afin de démontrer les différentes actions de l'agent de satiété selon la présente invention :

### Dosage 1

L'efficacité de l'agent de satiété contenant du safranal a été testée sur un panel de quatre personnes pendant une durée de 20 jours. Ces quatre personnes n'ont nullement modifié leurs habitudes alimentaires, ni leur mode de vie durant la durée de l'expérience. Elles ont ingéré quotidiennement en une prise au petit déjeuner 30 mg de l'extrait de safran.

Il a été ressenti une très nette diminution de la faim au déjeuner et au dîner. Une perte de poids en moyenne de 1 Kg, ainsi qu'une diminution du tour de taille de 1,2 cm et de 0,5 cm de tour de hanche pour chaque personne ont été constatées.

Ce test expérimental montre l'effet surprenant et inattendu du safranal sur la régulation de la faim, alors que le safran est usuellement utilisé en tant qu'arôme ou qu'adoucisseur de l'âpreté de certains aliments.

### Dosage 2 : test en clinique double aveugle vs placebo

Une seconde étude en clinique a été menée afin de démontrer l'effet satiétant des principes actifs du safran chez 16 sujets de sexe féminin sujets au grignotage compulsif. L'étude a été menée sur une durée de quatre semaines. Les femmes saines présentaient un léger surpoids, c'est-à-dire un IMC compris entre 22 et 30.

Une méthode de randomisation a été mise en place afin de répartir ces 16 sujets en deux groupes de 8. Un premier groupe (groupe 1) a pris chaque matin et midi, 2 gélules de placebo, tandis que le second groupe (groupe 2) a pris 2 gélules/j, également le matin et midi, d'une composition contenant un extrait de stigmates de safran, les deux groupes ayant chacun reçu des conseils diététiques.

La composition contenant un extrait de safran qui a été testé sur le groupe 2 comprenait entre autres par gélules de 267 mg +/- 10%, 90 mg d'extrait de stigmates de safran stabilisé sur support de cellulose microcristalline et silice colloïdale et acides gras riche en safranal, en crocine, en picrocrocine, 100 mg de maltodextrine et 2 mg de stéarate de magnésium. Plus particulièrement, chaque gélule contenait 0,9% de picrocrocine (soit 2,40 mg/gélule), 0,4% de safranal (soit 1,07 mg/gélule) et 0,3% de crocine (soit 0,80 mg/gélule).

Différents marqueurs d'efficacité tels que les mesures de poids, de masse grasse, masse hydrique et de l'IMC ont été mesurés, accompagnés d'une auto-évaluation de la sensation de faim, des quantité consommées pendant, entre, et après les repas. Cette auto-évaluation du sentiment de faim a été mesurée par le biais d'une évaluation subjective établie par une méthode visuelle numérique connue de l'homme de l'art.

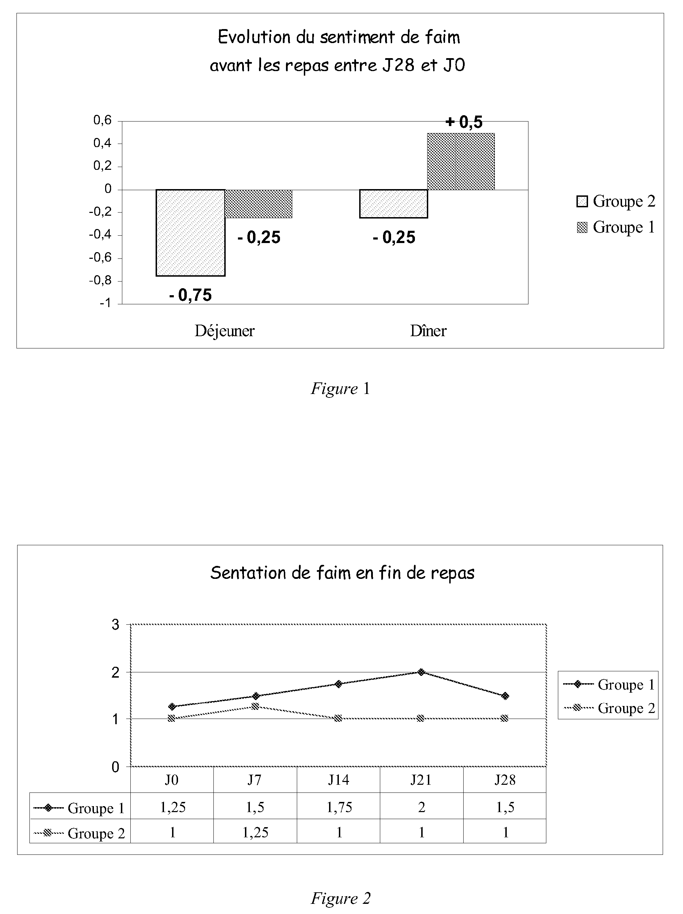

Tout d'abord comme l'illustre la figure 1 qui montre la mise en évidence du sentiment de faim avant les repas entre J28 et J0, les sujets du groupe 2 ont ressenti une plus forte diminution de la sensation de faim avant le déjeuner, qui persiste jusqu'au dîner, tandis que le groupe 1 (placebo) a ressenti un accroissement de la sensation de la faim.

En fin de repas, comme l'illustre la figure 2, le groupe 2 n'a pas ressenti de sensation de faim en fin de repas (pas de variations), tandis que le groupe 1 (placebo) a ressenti une augmentation de J0 à J28.

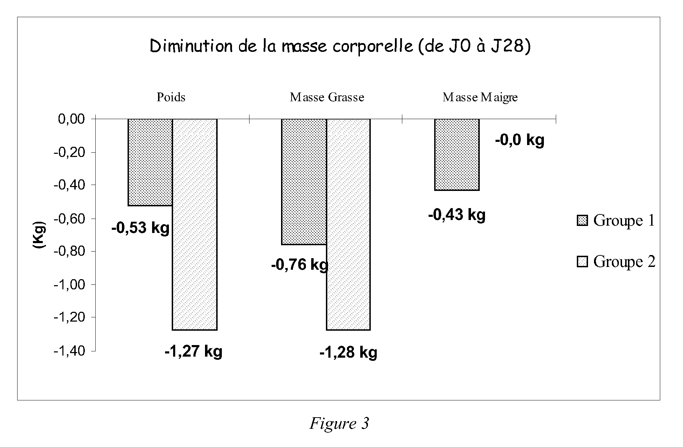

D'après la figure 3 qui montre la diminution de la masse corporelle de J0 à J28, après 28 jours de traitement de 2 gélules par jours matin et midi, une perte de poids en moyenne de -1,28 kg a été constaté dans le groupe 2 par rapport à une perte de poids en moyenne de -0,50 kg au sein du groupe 1 ; une perte de masse grasse en moyenne de -1,28 kg dans le groupe 2 par rapport au groupe placebo 1 de -0,17 kg ; et une perte de masse maigre de 0 kg au sein du groupe 2 par rapport au groupe placebo qui a perdu en moyenne -0,33 kg.

Ainsi, 100 % des femmes recevant des gélules comprenant les principes actifs du safran, c'est-à-dire une composition selon la présente invention, ont attesté avoir perçu une diminution de leur sensation de faim au moment du déjeuner et du dîner et ont déclaré avoir diminué leurs apports alimentaires pendant l'étude. A contrario, aucune des femmes sous placebo n'a constaté une diminution de ses apports alimentaires.

De plus, 25% des femmes recevant les gélules selon la présente invention ont qualifié leurs apports alimentaires de « très diminués » pendant l'étude.

D'ailleurs, la perte de poids la plus importante (-3,5kg) a bien été mesurée chez la femme qui a estimé ses apports alimentaires comme étant « très diminués » pendant l'étude.

La diminution des apports alimentaires dans le groupe 2 est ainsi reliée à une augmentation du sentiment de satiété qui contrairement à la restriction cognitive, n'induit pas de sentiment de frustration qui augmente le risque de reprise de poids.

En comparaison au groupe placébo (groupe 1), la supplémentation avec des gélules contenant un extrait de safran et par conséquent du safranal, de la crocine et de la picrocrocine, a favorisé la perte de poids, la perte de masse grasse corporelle tout en maintenant la masse maigre, donc la masse musculaire, stable.

La perte de poids moyenne est certes modeste mais sa significacité clinique reste probable du fait de l'absence de régime hypocalorique associé, les sujets ayant seulement reçu des conseils diététiques (par exemple, les grignotages avaient été déconseillés mais pas interdits) et la courte durée de l'étude par rapport à cette problématique.

Dans le cadre d'un régime hypocalorique : diminuer le sentiment de restriction pourrait améliorer la compliance au régime et la perte de poids associée ; ainsi que de réduire le risque de rebond, souvent lié à un relâchement consécutif à la période de régime.

A long terme (sans régime associé), améliorer la facilité à un sujet de gérer son comportement alimentaire peut se révéler intéressant lorsqu'on sait qu'éviter un excès de 20 Kcal/jour (soit l'équivalent d'un morceau de sucre) peut éviter une prise de poids de 1Kg de masse grasse en 1 an (Ancellin R., « Glucides et santé : état des lieux, évaluations et recommandations », 2003).

## Revendications

1. Utilisation d'un extrait de safran contenant du safranal, de la picrocrocine et de la crocine pour la production d'un agent de satiété actif pour le traitement des problèmes de santé liés à la surcharge pondérale.

2. Utilisation d'un extrait de safran contenant du safranal, de la picrocrocine et de la crocine pour la production d'un agent de satiété actif pour le traitement des troubles alimentaires, des envies compulsives de grignotage liées à la dépression.

3. Utilisation d'un extrait de safran contenant du safranal, de la picrocrocine et de la crocine pour la fabrication d'un agent de satiété actif pour le traitement de l'obésité.

4. Utilisation non thérapeutique d'un extrait de safran contenant du safranal, de la picrocrocine et de la crocine en tant qu'agent de satiété actif pour aider l'individu présentant une surcharge pondérale à réduire ou contrôler son absorption de calories au quotidien et/ou contrôler son poids et/ou son apparence physique.

5. Utilisation d'un extrait de safran contenant du safranal, de la picrocrocine et de la crocine selon l'une des revendications précédentes, dans laquelle ce ou ces principes actifs sont associés à au moins un excipient ou véhicule inerte, non toxique, pharmaceutiquement et/ou alimentairement acceptable permettant son administration par voie orale.

6. Utilisation d'un extrait de safran contenant du safranal, de la picrocrocine et de la crocine selon la revendication 5, dans laquelle ce ou ces principes actifs se présentent sous forme d'une solution ou d'une suspension aqueuse dans une ampoule en verre, en spray, en boisson, en flacon compte gouttes ou à l'état sec de comprimés nus ou enrobés, de gélules, de capsules, de poudres, de comprimés effervescents, de granules, de strips, de pastilles.

7. Utilisation d'un extrait de safran contenant du safranal, de la picrocrocine et de la crocine selon l'une des revendications précédentes, dans laquelle la teneur en safranal, picrocrocine et crocine est choisie pour une administration s'échelonnant entre 0,05 mg/jour et 180 mg/jour, de préférence entre 0,2 mg/jour et 10 mg/jour.

8. Utilisation d'un extrait de safran contenant du safranal, de la picrocrocine et de la crocine selon l'une des revendications précédentes, dans laquelle la teneur en safranal, picrocrocine et crocine au sein de l'agent de satiété est comprise entre 0,01 et 50 %, de préférence entre 0,05 et 20 %, et plus particulièrement entre 0,1 et 10 %, en poids, par rapport au poids total de l'agent de satiété lorsque celui-ci se trouve sous forme solide ou par rapport au volume de l'agent de satiété lorsque celui-ci se trouve sous forme liquide.

9. Utilisation d'un extrait de safran contenant du safranal, de la picrocrocine et de la crocine selon l'une des revendications précédentes, dans laquelle le safranal, la picrocrocine et la crocine sont extraits par hydrodistillation suivie d'extraction liquide/liquide ou par une hydrodistillation sous vide par micro-ondes (VMHD) ou encore par une extraction au CO2 supercritique, ou aux ultrasons, ou aux résines échangeuses d'ions avec des co-solvants et solvants d'élution polaires et/ou apolaires, ou encore par le jeu d'extraction solides/liquides suivies d'extractions liquides/liquides.

## Patentansprüche

1. Verwendung eines Safranextrakts, enthaltend Safranal, Picrocrocin und Crocin, für die Herstellung eines aktiven Sättigungsmittels zur Behandlung von Gesundheitsproblemen in Verbindung mit Übergewicht.

2. Verwendung eines Safranextrakts, enthaltend Safranal, Picrocrocin und Crocin, für die Herstellung eines aktiven Sättigungsmittels zur Behandlung von Ernährungsstörungen, von Heißhungerattacken in Verbindung mit Depression.

3. Verwendung eines Safranextrakts, enthaltend Safranal, Picrocrocin und Crocin, für die Herstellung eines aktiven Sättigungsmittels zur Behandlung der Fettleibigkeit.

4. Nicht therapeutische Verwendung eines Safranextrakts, enthaltend Safranal, Picrocrocin und Crocin, als aktives Sättigungsmittel zur Unterstützung von Personen mit Übergewicht bei der Reduzierung oder Kontrolle seiner täglichen Kalorienaufnahme und/oder Kontrolle seines Gewichts und/oder seines physischen Aussehens.

5. Verwendung eines Safranextrakts, enthaltend Safranal, Picrocrocin und Crocin, nach einem der vorangehenden Ansprüche, wobei dieser oder diese Wirkstoffe mit mindestens einem Hilfsstoff oder inerten, nicht toxischen, pharmazeutisch und/oder ernährungstechnisch akzeptablen Vehikel kombiniert sind, das seine Verabreichung auf oralem Weg erlaubt.

6. Verwendung eines Safranextrakts, enthaltend Safranal, Picrocrocin und Crocin, nach Anspruch 5, wobei dieser oder diese Wirkstoffe in Form einer Lösung oder einer wässrigen Suspension in einer Glasampulle, als Spray, Getränk, in einem Tropfenzählerflakon oder in trockenem Zustand in Form nackter oder ummantelter Tabletten, von Gelkapseln, Kapseln, Pulvern, Sprudeltabletten, Granulat, Strips, Pastillen vorliegen.

7. Verwendung eines Safranextrakts, enthaltend Safranal, Picrocrocin und Crocin, nach einem der vorangehenden Ansprüche, wobei der Gehalt an Safranal, Picrocrocin, und Crocin für eine Verabreichung ausgewählt ist, die zwischen 0,05 mg/Tag bis 100 mg/Tag, vorzugsweise zwischen 0,2 mg/Tag und 10 mg/Tag gestaffelt ist.

8. Verwendung eines Safranextrakts, enthaltend Safranal, Picrocrocin und Crocin, nach einem der vorangehenden Ansprüche, wobei der Gehalt an Safranal, Picrocrocin und Crocin innerhalb des Sättigungsmittels zwischen 0,01 und 50 Gew.-%, vorzugsweise zwischen 0,05 und 20 Gew.-% und insbesondere zwischen 0,1 und 10 Gew.-% inklusive in Bezug zum Gesamtgewicht des Sättigungsmittels beträgt, wenn dieses in fester Form vorliegt, oder in Bezug zum Volumen des Sättigungsmittels, wenn dieses in flüssiger Form vorliegt.

9. Verwendung eines Safranextrakts, enthaltend Safranal, Picrocrocin und Crocin, nach einem der vorangehenden Ansprüche, wobei das Safranal, das Picrocrocin und das Crocin mittels Hydrodestillation, gefolgt von Extraktion Flüssigkeit/Flüssigkeit, oder mittels einer Hydrodestillation im Vakuum mittels Mikrowellen (VMHD) oder auch mittels einer Extraktion mit superkritischem CO₂ oder mit Ultraschall oder mit Ionentauscherharzen mit polaren und/oder apolaren Elutionsco-Lösungsmitteln und Elutionslösungsmitteln oder auch mittels Extraktionskombination Feststoffe/Flüssigkeiten, gefolgt von Extraktionen Flüssigkeiten/Flüssigkeiten, extrahiert werden.

## Claims

1. The use of a saffron extract containing safranal, picrocrocin and crocin for the production of an active satiety agent to treat health problems related to overweight.

2. The use of a saffron extract containing safranal, picrocrocin and crocin for the production of an active satiety agent to treat eating disorders, compulsive snacking related to depression.

3. The use of a saffron extract containing safranal, picrocrocin and crocin for the production of an active satiety agent to treat obesity.

4. The non-therapeutic use of a saffron extract containing safranal, picrocrocin and crocin as active satiety agent to help overweight individuals to reduce or control their daily calorie intake and/or to control their weight and/or their physical appearance.

5. The use of a saffron extract containing safranal, picrocrocin and crocin according to one of the preceding claims, wherein this or these active ingredients are associated with at least one excipient or inert carrier that is nontoxic, pharmaceutically and/or palatably acceptable allowing administration via oral route.

6. The use of a saffron extract containing safranal, picrocrocin and crocin according to claim 5, wherein this or these active ingredients are in the form of a solution or aqueous suspension in a glass vial, in a spray, beverage, in a dropper bottle or in the dry state of coated or uncoated tablets, hard capsules, soft capsules, powders, effervescent tablets, granules, strips or lozenges.

7. The use of a saffron extract containing safranal, picrocrocin and crocin according to one of the preceding claims, wherein the content of safranal, picrocrocin and crocin is selected for administration ranging between 0.05 mg/day and 180 mg/day, preferably between 0.2 mg/day and 10 mg/day.

8. The use of a saffron extract containing safranal, picrocrocin and crocin according to one of the preceding claims, wherein the content of safranal, picrocrocin and crocin in the satiety agent is between 0.01 and 50 %, preferably between 0.05 and 20 % and more particularly between 0.1 and 10 % by weight relative to the total weight of the satiety agent when it is in solid form or relative to the volume of the satiety agent when it is in liquid form.

9. The use of a saffron extract containing safranal, picrocrocin and crocin according to one of the preceding claims, wherein the safranal, picrocrocin and crocin are extracted via hydrodistillation followed by liquid/liquid extraction, or via vacuum microwave hydrodistillation (VMHD), or via extraction using supercritical CO2 or ultrasound or ion exchange resins with polar and/or apolar eluting co-solvents and solvents, or via solid/liquid extraction followed by liquid/liquid extraction.
